(19) Europäisches Patentamt
European Patent Office
Office européen des brevets

(11) EP 4 649 965 A1

(12) **EUROPEAN PATENT APPLICATION**

(43) Date of publication:
**19.11.2025 Bulletin 2025/47**

(21) Application number: **24175611.3**

(22) Date of filing: **14.05.2024**

(51) International Patent Classification (IPC):
***A61K 49/00*** *(2006.01)*

(52) Cooperative Patent Classification (CPC):
**A61K 49/0032; A61K 49/0056**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Oncopole Claudius Regaud**
**31059 Toulouse Cedex 9 (FR)**

(72) Inventors:
- **PAQUEREAU, Laurent**
  **31450 Baziège (FR)**
- **FERRON, Gwenaël**
  **31000 Toulouse (FR)**
- **COUSTETS, Mathilde**
  **31320 Castanet-Tolosan (FR)**

(74) Representative: **Barbot, Willy**
**Simodoro-ip**
**82, rue Sylvabelle**
**13006 Marseille (FR)**

Remarks:
The complete document including Reference Table(s) and the Sequence Listing(s) can be downloaded from the EPO website

(54) **COMPOSITION FOR THE TARGETED IMAGING OF CARCINOMA**

(57) The present invention concerns a new pharmaceutical composition with less than 10 EU (endotoxin unit) per mL of composition (UE/mL) and comprising an imaging agent consisting of a conjugate of 1) a polypeptide A38C having the amino acid sequence SEQ ID NO: 1 with 2) a near Infra-red (IR) fluorophore, and its uses for performing image guided diagnosis and surgery respectively on a subject.

EP 4 649 965 A1

## Description

### Technical field

The present invention relates to the field of cancer, and composition and methods for use therein.

### Background

Cancer currently accounts for approximately 13% of all deaths worldwide. Despite decades of intense research, cancer remains the second leading cause of death in the highly developed countries, accounting for nearly 25% of the deaths. For many types of cancer, surgery - often in combination with chemotherapy, radiation treatment or hyperthermia - is the mainstay of therapeutic intervention. Now, it was demonstrated that the complete surgical resection is a critical factor for improving the patient's prognosis. This maximal cytoreduction strategy is part of therapeutic protocols that include systemic or intraperitoneal chemotherapy possibly associated with hyperthermia.

Among cancers, carcinoma is a generic term including all cancers originating from the epithelial tissue. Those solid tumors represent the most common form of cancer and a major cause of lethality. Epithelial ovarian cancer (EOC) is a carcinoma with a relatively low incidence compared to other cancers in women. Now, EOC has a strong lethality due to the lack of symptoms and of reliable diagnosis methods at early stages of the disease. As a result, over 75% of EOC cases are diagnosed at advanced stages. Those advanced stages -i.e., IIb to IV in FIGO (International Federation of Gynecology and Obstetrics) classification- are concomitant with the cancer cells spreading from the primary tumor site. Such spreading mainly occurs throughout the peritoneal cavity resulting in poor prognosis peritoneal carcinomatosis (PC). This metastasis process is atypical because clusters of cells are driven to their implantation sites by peritoneal flow. Despite improvements during the last decade in five-year overall survival, most of the patients are still subjected to EOC relapses, said relapses occurring inside the peritoneal cavity.

Surgery is still the first therapy for all the solid tumors including carcinomas. While surgery is effective in 50 percent of patients with solid tumors in the US, chemo- and radiotherapy alone are effective in less than 5 percent of all cancer patients. Thus, the standard of care for managing patients diagnosed with ovarian PC is a cytoreductive surgery as complete as possible followed by intraperitoneal and/or systemic chemotherapy. Here lies a major challenge since it is indispensable to remove every residuals cancerous cells from the peritoneal cavity to prevent tumor recovery. In fact, peritoneal metastasis implants are variable in size, ranging from microscopic nodules to several centimeters' tumor masses. Nevertheless, differentiation between tumor and normal tissues is not always easy, especially when patients have been given neoadjuvant treatments.

*In situ* photodetection was initially envisaged for improving cancer resection surgery. The principle of photodetection is based on the use of a selected compound having affinity to a tumor and being able to be optically visualized at a selected wavelength of light for helping the surgeon to identify the potentially affected tissues. Whereas photodetection of tumors was originally developed in the 1980s, very few protocols using photodetection are currently used in the hospital. While some probes using specific dyes have been used to target some tumours, the resulting tumour-to-background ratios -i.e., Signal-to-Noise Ratio (SNR)- are often poor and the boundaries between malignant and healthy tissues is generally difficult to define. The different probes developed and used for fluorescence-guided surgery are listed in the table 1 of MANGEOLLE et al. (Nanomaterials, vol.8(8): 572, doi: 10.3390/nano8080572, 2018). These probes include Indocyanine green (SNR=2.2, passive probe used in the clinic despite 62% false positives) and OTL38 (CYTALUX, SNR=4.4). The Signal-to-Noise Ratio (SNR) of these probes being less than 7 (even less than 5) corresponds to difficulties for performing image guided surgery. The inventors have previously designed and characterized the probe A38C-ALEXA647 in an ovarian peritoneal carcinomatosis mouse model (COUSTETS et al., Biomaterial, vol.241:119908, doi: 10.1016/j.biomaterials.2020.119908, 2020). Now, the A38C-ALEXA647 SNR was comprised between 4 and 7. Those values are certainly interesting but they only position the A38C-ALEXA647 probe as a starting point among others for developing a further probe having the required sensitivity and specificity to be used in therapy.

In conclusion, because of the low SNR associated with known probes, the authors of MANGEOLLE *et al.* (abovementioned, 2018) suggest that the ideal probe for fluorescence-guided surgery having the required sensitivity and specificity should be multimodal by associating a near infrared dye, with another imaging agent for either computed tomography (CT) or magnetic resonance imaging (MRI) to overcome the lack of specificity and limitation of fluorescence depth of detection. In the meantime, the residual microscopic cancer nodules, undetectable to the naked eye, are still often the cause of relapses after cytoreductive surgery and remain despite chemotherapy.

### Summary of the invention

The inventors have now developed a new ovarian peritoneal carcinomatosis mouse model using patients' xenografts. They have used the A38C-alexa647 probe for validating this model as compared to their previously disclosed

ovarian peritoneal carcinomatosis mouse model (COUSTETS et al., abovementioned, 2020).

Now and surprisingly, the inventors established that the SNRs of the A38C-ALEXA647 probe was much more important in this patients' derived xenograft (PDX) model -i.e., 7 to 14- than those observed in the previously disclosed mouse model in COUSTETS *et al.* (abovementioned, 2020; with SNRs from 4 to 7). PDX models being much more representative of *in vivo* tumor environment and clinic situations, such unexpected results thus teach that the A38C-ALEXA647 probe on its own can be used in cytoreductive surgery without any significative lack of specificity nor without limitation of fluorescence depth of detection (because of the SNRs comprised between 7 and 14).

The SNRs observed in the PDX model are associated with an increase of apparent affinity of the A38C-ALEXA647 probe as compared to the apparent affinity previously observed. Afterwards, this increase of apparent affinity was correlated with an added step of anion-exchange chromatography (AEX) using SARTOBIND Q resulting in endotoxins elimination. Finally, the decreased endotoxins level in the A38C-ALEXA647 probe's composition seems to be correlated to the increased apparent affinity of the purified A38C-ALEXA647 probe's composition. The endotoxins may have interfered with the conjugate structure, thus affecting its apparent affinity.

Accordingly, a first object of the invention is directed to a pharmaceutical composition comprising an imaging agent, eventually associated with a pharmaceutically acceptable carrier, consisting in a conjugate of:

a) a polypeptide A38C having the amino acid sequence SEQ ID NO: 1, and
b) a Near Infra-red (NIR) fluorophore;

wherein the endotoxin concentration of said pharmaceutical composition is less than 10 International Units (IU) of endotoxins per mL of composition, preferably less than 5 IU/mL, and most preferably less than 1 IU/mL.

Because of this unexpected important SNRs, the A38C-ALEXA647 probe can be used in diagnosis and in therapy by its own, without being combined with any other imaging agent like either computed tomography (CT) or magnetic resonance imaging (MRI) to overcome the lack of specificity and limitation of fluorescence depth of detection as suggested by MANGEOLLE *et al.* (abovementioned, 2018).

Thus, in a first preferred embodiment, the pharmaceutical composition of the invention does not comprise any other imaging agent -i.e., other than the NIR fluorophore of the conjugate-.

A second object of the invention relates to a composition comprising the conjugate as defined previously for use in a method of performing image guided surgery of tumoral tissues on a subject suffering from carcinoma comprising:

a) illuminating the conjugate to visualize the near infrared fluorophore's conjugate using a corresponding infrared light; and
b) performing surgical resection of the tumoral tissues that fluoresce upon excitation by the infrared light, wherein the subject has been administered with said composition under conditions and for a time sufficient for the conjugate to accumulate at a given surgical site.

A third object of the invention relates to the composition comprising the conjugate as defined previously for use in a method of performing image guided carcinoma diagnosis on a subject comprising:

a) illuminating the conjugate to visualize the near infrared fluorophore's conjugate using a corresponding infrared light; and
b) performing carcinoma diagnosis, when identifying tumoral tissues that fluoresce upon excitation by the infrared light, wherein the subject has been administered with said composition under conditions and for a time sufficient for the conjugate to accumulate at a given surgical site.

According to a first preferred embodiment of the methods of the invention, the subject has not been administrated with any other imaging agent -i.e., other than the conjugate-.

The inventors previously detect residual microscopic tumor nodules undetectable to the naked eye (< 1 mm2; COUSTETS *et al.,* abovementioned, 2020). Because of the A38C-ALEXA647 probe's SNRs now comprised between 7 and 14, the inventors were now able to increase considerably this detection threshold by identifying carcinoma nodules whose surface was as few as 50 $\mu m^2$. Because of this considerable increase in this detection threshold, the methods of the invention thus present an increased sensitivity. Accordingly, the risk of patients' relapses will be reduced, thus increasing the life expectancy of the patients.

According to a second preferred embodiment of the methods of the invention, the tumoral tissues are tumoral nodules whose surface is less than 10 000 $\mu m^2$ -i.e., 0.01 $mm^2$- corresponding to the detection threshold in COUSTETS *et al.* (abovementioned, 2020), preferably carcinoma nodules whose surface is equal or less than 100 $\mu m^2$; and preferably carcinoma nodules whose surface is around 50 $\mu m^2$.

More, the inventors have established that the SNRs values of the A38C-ALEXA647 probe for EOC tumoral

tissues located in the digestive tract, in the large omentum and in the retroperitoneum in this patients' derived xenograft (PDX) model were of 7, 9 and 14 respectively, instead of the SNRs values of 4, 5 and 6 respectively in the IGROV model. Accordingly, these results teach that unexpectedly the A38C-ALEXA647 can be used directly and efficiently for locating such EOC tumoral tissues at these locations for both surgery and diagnosis.

According to a third preferred embodiment of the methods of the invention, the carcinoma is an Epithelial ovarian cancer (EOC), preferably the tumoral tissues are EOC nodules located in the digestive tract, in the large omentum, and/or in the retroperitoneum.

**Detailed description**

As used herein, the term "endotoxins" refer to lipopolysaccharides found in the outer membrane of Gram-negative bacteria. They are large molecules consisting of a lipid and a polysaccharide composed of O-antigen, outer core and inner core joined by a covalent bond. They are released only during the lysis of these bacteria, and can cause a disproportionate general inflammatory response, or systemic inflammatory response syndrome, which can lead to death. If endotoxin reaches the bloodstream, it can lead to septic shock.

Endotoxins from gram-negative bacteria are the most common cause of toxic reactions resulting from contamination of pharmaceutical products with pyrogens; their pyrogenic activity is much higher than that of most other pyrogenic substances. Although there are a small number of pyrogens which possess a different structure, the conclusion is generally justified that the absence of bacterial endotoxins in a product implies the absence of pyrogenic components, provided the presence of non-endotoxin pyrogenic substances can be ruled out.

Endotoxin concentration is expressed in International Unit (IU) of endotoxin per mL. Now, one International Unit (IU) of endotoxin is equal to one Endotoxin Unit (EU). One EU/mL equals approximately 0.1 to 0.2 ng endotoxin/mL of solution.

Methods for detecting and quantifying endotoxins are known from the skilled person (European pharmacopoeia 2.6.14-D) and are based on the following 3 techniques: 1) the gel-clot technique, which is based on gel formation; 2) the turbidimetric technique, based on the development of turbidity after cleavage of an endogenous substrate; and 3) the chromogenic technique, based on the development of color after cleavage of a synthetic peptide-chromogen complex.

Such methods use amoebocyte lysate from horseshoe crab (*Limulus polyphemus* or *Tachypleus tridentatus*) and include Gel-clot method - limit test; Gel-clot method - semi-quantitative test; Turbidimetric kinetic method; Turbidimetric end-point method; Chromogenic kinetic method; and Chromogenic end-point method.

The gel-clot technique allows detection or quantification of endotoxins and are based on clotting of the lysate in the presence of endotoxins. The concentration of endotoxins required to cause the lysate to clot under standard conditions is the labelled lysate sensitivity.

The turbidimetric technique is a photometric test to measure the increase in turbidity. Based on the test principle employed, this technique is classified as being the end-point-turbidimetric method or the kinetic-turbidimetric method. The end-point-turbidimetric method is based on the quantitative relationship between the endotoxin concentration and the turbidity (absorbance or transmission) of the reaction mixture at the end of an incubation period. The kinetic-turbidimetric method is based on the measure either of the time (onset time) needed for the reaction mixture to reach a predetermined absorbance, or the rate of turbidity development. Both methods are carried out at the incubation temperature recommended by the lysate manufacturer (usually 37 ± 1 °C).

The chromogenic technique is based on the measure of chromophore released from a suitable chromogenic peptide by the reaction of endotoxins with the lysate. The end-point-chromogenic method is based on the quantitative relationship between the endotoxin concentration and the quantity of chromophore released at the end of an incubation period. The kinetic-chromogenic method measures either the time (onset time) needed for the reaction mixture to reach a predetermined absorbance, or the rate of color development. Again, these methods are carried out at the incubation temperature recommended by the lysate manufacturer (usually 37 ± 1 °C).

Preferably, the method used for detecting and quantifying endotoxins is the chromogenic kinetic method. As an example, the endotoxins detection and quantification are done using ENDOSAFE NEXTGEN-PTS (CHARLES RIVER).

As used herein, the term "fluorophore" reverts to is a fluorescent chemical compound that can re-emit light upon light excitation.

Now, the NIR fluorophore does not constitute a diagnostic agent by itself. Indeed, the NIR fluorophore administered alone -i.e., not conjugated with the polypeptide A38C-does not make it possible to identify whether a subject is affected or likely to develop a carcinoma.

Fluorophores are well known from the skilled person and include xanthene, rhodamine, cyanine, squaraine, naphthalene, coumarin, oxadiazole, anthracene, pyrene, oxazine, acridine, arylmethine, tetrapyrrole, and dipyrromethene based dyes.

Commercial fluorophores include CF dye (BIOTIUM), DRAQ and CYTRAK probes (BIOSTATUS), BODIPY (INVITROGEN), EVERFLUOR (SETAREH BIOTECH), ALEXA FLUOR (INVITROGEN), BELLA Fluor (SETAREH

BIOTECH), DYLIGHT Fluor (THERMO SCIENTIFIC, PIERCE), ATTO AND TRACY (SIGMA ALDRICH), FLUOPROBES (INTERCHIM), ABBERIOR Dyes (ABBERIOR), DY and MEGASTOKES Dyes (DYOMICS), SULFO CY dyes (CYANDYE), HILYTE Fluor (ANASPEC), SETA, SETAU and SQUARE Dyes (SETA BIOMEDICALS), QUASAR and CAL FLUOR dyes (BIOSEARCH TECHNOLOGIES), SURELIGHT Dyes (APC, RPEPERCP, PHYCOBILISOMES) (COLUMBIA BIOSCIENCES), APC, APCXL, RPE, BPE (PHYCO-BIOTECH, GREENSEA, PROZYME, FLOGEN), and VIO Dyes (MILTENYI BIOTEC).

As used herein, a "Near Infra-red (NIR) fluorophore" is a fluorophore, whose absorption and emission wavelengths are greater than 600 nm. Typically, said absorption and emission wavelengths are both comprised between 600 and 1750 nanometers. Now, because of the, the combination of light absorption by hemoglobin in the visible light spectrum (<600 nm) and water and lipids in the IR range (>900 nm), it is preferable to select an optical imaging window from approximately 600 to 900 nm, and below 1,000 nm in which the absorption coefficient of tissues is at a minimum.

Moreover, light in the near infrared region induces very little autofluorescence and permeates tissue much more efficiently. Another benefit to near-IR fluorophore is that the background from the scattered light from the excitation source is greatly reduced since the scattering intensity is proportional to the inverse fourth power of the wavelength.

As used herein, a "Near Infra-red (NIR) fluorophore" is a strict Near Infra-Red (NIR) fluorophore or a NIR-II fluorophore, preferably a strict Near Infra-Red (NIR) fluorophore. Strict Near Infra-Red (NIR) fluorophores are fluorophores whose absorption and emission wavelengths are comprised between 600 and 900 nm, preferably between 650 to 850 nm. Now, the NIR-II fluorophores are fluorophores whose absorption and emission wavelengths are comprised between 1 000 and 1 750 nm.

Such Near Infra-Red (NIR) fluorophores are well known from the skilled person and can be simply identified. Such Near Infra-Red (NIR) fluorophores can be identified on THERMOFISHER website (*See.* https://www.thermofisher.com/fr/fr/home/life-science/cell-analysis/fluorophores.html).

As an example of such Near Infra-Red (NIR) fluorophore, one can cite INDOCYNINE GREEN (ICG), IRDYE800CW, ZW-800-1, SO456, Sulfo-Cy5 and Sulfo-Cy5.5, BM104 and BM105 (the NHS-activated version of BM104), CH1055, Allophycocyanin (APC), NOVAFLUOR 660, Cyanine5 (Cy5), EFLUOR660, ALEXA FLUOR 647, NOVAFLUOR RED 685, NOVAFLUOR RED 690, ALEXA FLUOR 660, NOVAFLUOR RED 700, ALEXA FLUOR 680, NOVAFLUOR RED 710, ALEXA FLUOR 700, NOVAFLUOR RED 725, NOVAFLUOR RED 755, ALEXA FLUOR 750, APC-EFLUOR 780, squaraines, xanthene derivatives, and boron dipyrromethenes (BODIPYs) or aza-BODIPYs.

The *Xerocomus chrysenteron* lectin (XCL) belongs to a group of proteins selectively recognizing the Thomsen-Friedenreich antigen (TF antigen, T antigen or CD176). This T antigen corresponds to a disaccharide (β-D-Gal (1-3) D-GalNAc) highly expressed by about 80-90% of human carcinomas, which is rare in normal epithelia. The binding of XCL to the TF antigen expressed by membrane proteins presents a high affinity with dissociation constants of 1.6 μM and 0.38 μM for fetuin and asialofetuin, respectively. After its binding on cell surface markers, XCL was quickly endocytosed by a clathrin-dependent mechanism to end up in late endosomes/lysosomes. This cellular binding was totally blocked in the presence of a competitive sugar highlighting the need for TF antigen binding to achieve cellular uptake of the protein. The 3D structure of the XCL protein was resolved by X-ray crystallography and revealed a homo-tetrameric assembly. For stabilizing this structure, the inventors previously designed recombinant XCL proteins comprising disulfide bonds for preventing the dissociation of oxidized tetramers. The distance between the Cβ of alanine 38 and its counterpart is 3.4 Angstroms, which is a typical range for Cβ of cysteines involved in a disulfide bridge. Accordingly, the A38C variant (SEQ ID NO:1) has been produced, wherein the alanine 38 has been substituted by a cysteine (LADURANTIE et al., Nanoscale, vol.11, p:3248-3260, 2019). Finally, this A38C variant was demonstrated as being more stable than the XCL wild type protein under the tetrameric form, and as having a complete oxidation and reduction capacity. It will also be understood that the natural amino acids of SEQ ID NO: 1 may be replaced by chemically modified amino acids, potentially for increasing the polypeptide stability.

Advantageously, the A38C is under a multimeric form corresponding to a tetramer.

As demonstrated in LADURANTIE et al. (Nanoscale, vol. 1 1, p:3248-3260, 2019), the A38C-ALEXA647 conjugate is a tetramer, that can split into two dimers only upon reduction of the two disulfide bridgesThus, after its binding to Thomsen-Friedenreich antigen (TF antigen), the conjugate is internalized into the lysosomal compartments, wherein the structure of the polypeptide A38C is modified from a tetrameric to a dimeric form following the reduction of its two disulfide bridges in the reductive lysosomal environment.

Because of the acidic environment of lysosomal compartments -i.e., pH 4.5 to 5-the selected NIR fluorophore is preferably a stable dye under acidic environment. As used herein, an acid stable NIR fluorophore is a fluorophore, whose structure and fluorescence are both maintained at pH 4.5 to 5.0 -as compared to pH 7.0 to 7.5-.

Such acid stable Near Infra-red (IR) fluorophore can be simply identified by the skilled person on the basis of its general knowledge.

As an example of such near Infra-red (IR) fluorophore stable in the acidic lysosomal environment, we can cite INDOCYNINE GREEN (ICG), IRDYE800CW, ZW-800-1, SO456, Sulfo-Cy5 and Sulfo-Cy5.5, BM104 and BM105 (the NHS-activated version of BM104), CH1055, boron dipyrromethenes (BODIPYs) or aza-BODIPYs, and ALEXA FLUOR

647.

Advantageously, the fluorophore is ALEXA FLUOR 647.

ALEXA FLUOR 647 dye is a bright, far-red-fluorescent dye with excitation ideally suited for the 594 nm or 633 nm laser lines. The structure of ALEXA FLUOR 647 is as follows.

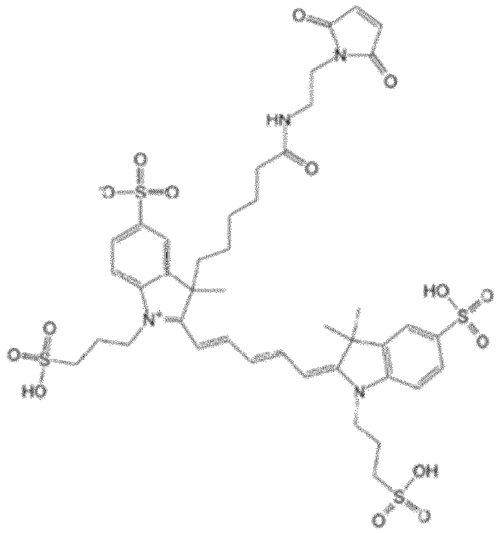

The term "conjugate" is used in its general meaning in the art and refers to a covalent or non-covalent complex.

The conjugate concentration for the composition of the invention is comprised between 0.1 and 100 mg/mL (mg of conjugate per mL of composition), preferably between 0.5 and 5 mg, and most preferably around 1 mg/mL.

The typical labelling ratio of the conjugate of the invention is comprised between 0.5 and 4 moles of NIR fluorophore per mole of A38C tetramer, preferably between 0.5 and 2 moles of NIR fluorophore per mole of A38C tetramer, and most preferably around 1 mole of NIR fluorophore per mole of A38C tetramer.

According to a preferred embodiment, the term conjugate refers to a covalent complex between:

A) a polypeptide A38C having the amino acid sequence SEQ ID NO: 1, and
B) a NIR fluorophore;

Such covalent complex can be simply obtained by the skilled person according to his general knowledge using fluorophore protein labelling agents and kits. The identification of defined fluorophore labelling agents and kits can be done for example on the THERMOFISHER website dedicated to fluorophore (https://www.thermofisher.com/fr/fr/home/life-science/cell-analysis/fluorophores.html) providing wavelength excitation, wavelength emission, chemical properties, platform, protein Labelling Reagents Selection Guide and fluorophore conjugated antibodies for a complete list of fluorophores.

Still preferably, the conjugate is a covalent complex between the A38C variant (SEQ ID NO: 1) and the ALEXA FLUOR 647 dye.

Such conjugate can be obtained by incubating the A38C variant with an ALEXA FLUOR 647 NHS ester as described in the Examples. As an example of commercially available ALEXA FLUOR 647 NHS ester, one can cite the one commercialized by MEDCHEMEXPRESS (cat n° Cat. No.: HY-D2096) or by THERMOFISCHER SCIENTIFIC (Catalog number: A20006) for example.

According to a preferred embodiment, the pharmaceutical composition of the invention does not comprise any other imaging agent -i.e., other than the NIR fluorophore of the conjugate-.

As used herein, an "imaging agent" refers to a contrast agent that can be administered to a patient and that can emit energy through fluorescence, X-rays, gamma rays, sound waves, radio waves, and radioactive particles.

As an example of such imaging agent, one can cites, fluorescence imaging agents -i.e., fluorophores-, radiocontrast imaging agents used in emission tomography (PET), in computed tomography (CT) or in magnetic resonance imaging (MRI).

The expression "pharmaceutically acceptable" refers to molecular entities and compositions that are physiologically tolerable and do not typically produce allergic or similar undesirable reactions, such as gastric upset, dizziness and the like when administered to a human. Preferably, as used herein, the expression "pharmaceutically acceptable" means approvable by a regulatory agency of the Federal or state government or listed in the U.S. Pharmacopeia or other generally recognized pharmacopeia for use in animals, and more particularly in humans.

The term "carrier" refers to a solvent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by E.W. Martin.

The conjugate may be solubilized in a buffer or water or incorporated in emulsions, microemulsions, hydrogels (e.g. PLGA-PEG-PLGA triblock copolymers-based hydrogels), in microspheres, in nanospheres, in microparticles, in nanoparticles (e.g. poly(lactic-co-glycolic acid) microparticles (e.g. poly lactic acid (PLA) ; poly (lactide-co-glycolic acid) (PLGA) ; polyglutamate microspheres, nanospheres, microparticles or nanoparticles), in liposomes, or other galenic formulations. In all cases, the formulation must be sterile and fluid to the extent of acceptable syringeability. It must be stable under the conditions of manufacture and storage and must be preserved against the contaminating action of microorganisms, such as bacteria and fungi.

Dispersions can also be prepared in glycerol, liquid polyethylene glycols, mixtures thereof and in oils. Under ordinary conditions of storage and use, these preparations contain a preservative to prevent the growth of microorganisms.

The conjugate can be formulated into a composition in a neutral or salt form. Pharmaceutically acceptable salts include the acid addition salts (formed with the free amino groups of the protein) which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or a dispersion medium containing, for example, water, ethanol, polyol (for example, glycerol, propylene glycol, and liquid polyethylene glycol, and the like), suitable mixtures thereof, and vegetables oils. The conjugates of the invention may also be modified, by pegylation as an example, so as to increase its biodisponibility.

The proper fluidity can be maintained, for example, using a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride.

The pharmaceutical composition contains vehicles which are pharmaceutically acceptable for the selected administration. In relation to a composition intended to be injected, the vehicles may be isotonic, sterile, saline solutions (monosodium or disodium phosphate, sodium, potassium, calcium, or magnesium chloride and the like or mixtures of such salts), or dry, especially freeze-dried compositions which upon addition, depending on the case, of sterilized water or physiological saline, permit the constitution of injectable solutions.

Prolonged absorption of the injectable compositions can be brought about by the use in the compositions of agents delaying absorption, for example, aluminium monostearate, gelatin, polyols, half-life enhancing covalent and non-covalent formulations.

There are numerous causes of peptide instability or degradation, including hydrolysis and denaturation. Hydrophobic interaction may cause clumping of molecules together (i.e., aggregation). Stabilizers may be added to reduce or prevent such problems.

Stabilizers include cyclodextrin and derivatives thereof (see U.S. Pat. No.5,730,969). Suitable preservatives such as sucrose, mannitol, sorbitol, trehalose, dextran and glycerin can also be added to stabilize the final formulation. A stabilizer selected from ionic and non-ionic surfactants, D-glucose, D-galactose, D-xylose, D-galacturonic acid, trehalose, dextrans, hydroxyethyl starches, and mixtures thereof may be added to the formulation. Addition of alkali metal salt or magnesium chloride may stabilize a peptide. The peptide may also be stabilized by contacting it with a saccharide selected from the group consisting of dextran, chondroitin sulphuric acid, starch, glycogen, dextrin, and alginic acid salt. Other sugars that can be added include monosaccharides, disaccharides, sugar alcohols, and mixtures thereof (E.g., glucose, mannose, galactose, fructose, sucrose, maltose, lactose, mannitol, xylitol). Polyols may stabilize a peptide and are water-miscible or water-soluble. Suitable polyols may be polyhydroxy alcohols, monosaccharides and disaccharides including mannitol, glycerol, ethylene glycol, propylene glycol, trimethyl glycol, vinyl pyrrolidone, glucose, fructose, arabinose, mannose, maltose, sucrose, and polymers thereof. Various excipients may also stabilize peptides, including serum albumin, amino acids, heparin, fatty acids and phospholipids, surfactants, metals, polyols, reducing agents, metal chelating agents, polyvinyl pyrrolidone, hydrolysed gelatin, and ammonium sulfate.

A second object of the invention relates to the composition comprising the conjugate as defined previously for use in a method of performing image guided surgery of tumoral tissues on a subject suffering from carcinoma comprising:

a) illuminating the conjugate to visualize the near infrared fluorophore's conjugate using a corresponding infrared light; and

b) performing surgical resection of the tumoral tissues that fluoresce upon excitation by the infrared light, wherein the subject has been administered with said composition under conditions and for a time sufficient for the conjugate to accumulate at a given surgical site.

A third object of the invention relates to the composition comprising the conjugate as defined previously for a further use in a method of performing carcinoma image guided diagnosis on a subject comprising:

a) illuminating the conjugate to visualize the near infrared fluorophore's conjugate using a corresponding infrared light; and
b) performing carcinoma diagnosis, when identifying tumoral tissues that fluoresce upon excitation by the infrared light, wherein the subject has been administered with said composition under conditions and for a time sufficient for the conjugate to accumulate at a given surgical site.

As mentioned previously, the unexpected important SNRs of the A38C-alexa647 probe enable this probe to be used in diagnosis and in therapy by its own, without being combined with any other imaging agent like either computed tomography (CT) or magnetic resonance imaging (MRI) to overcome the lack of specificity and limitation of fluorescence depth of detection of the known probes.
Thus and according to a first preferred embodiment of the methods of the invention, the subject has not been administrated with any other imaging agent -i.e., other than the conjugate comprising the near-IR fluorophore -.
As used herein, a carcinoma refers to cancer that develops from epithelial cells. Specifically, a carcinoma is a cancer that begins in a tissue that lines the inner or outer surfaces of the body, and that arises from cells originating in the endodermal, mesodermal or ectodermal germ layer during embryogenesis.
In a preferred embodiment, said carcinoma expressed the Thomsen-Friedenreich antigen (TF antigen, T antigen or CD 176). As used herein, the T antigen refers to a disaccharide (β-D-Gal (1-3) D-GalNAc) highly expressed by about 80-90% of human carcinomas, which is rare in normal epithelia (SPRINGER; J. Mol. Med., vol.75, p:594-602, 1997).
In another preferred embodiment, said carcinoma is an Epithelial ovarian cancer (EOC), preferably said EOC is a stage IIb to IV EOC.
Advantageously, the tumoral tissues are EOC nodules located in the digestive tract, in the spleen/large omentum, and/or in the retroperitoneum.
In this manner, the composition of the invention can be used for the *in vivo* identification of tumoral tissues in a subject in need thereof. The disclosed methods of the invention include illuminating, with a light having at least one excitation wavelength in the near infrared range from about 600 nm to about 900 nm, an *in vivo* body part of the subject potentially containing tumoral tissue. In fact, the fluorescence emanating from the NIR fluorophore of the conjugate corresponds to conjugates internalized in the lysosome of the tumoral cells. Therefore, fluorescence emitted by the internalized near infrared fluorophore will be clearly visible and distinct from the auto-fluorescence of the surrounding tissue.
The excitation light used for illuminating step may be monochromatic or polychromatic. Operating rooms for surgical procedures are already equipped with an overhead light that produces wavelengths of light in the optical emitting spectrum useful in practice of methods of the invention, such as a lamp that produce light in the appropriate wavelength. Now, an endoscopic device can be used to deliver the excitation light to the site, to receive fluorescence emanating from the site within a body cavity, and to aid in formation of a direct image of the fluorescence from the tumoral tissue. For example, a lens in the endoscopic device can be used to focus the detected fluorescence as an aid in formation of the image. Such an excitation light can be utilized in the practice of the methods of the invention merely by turning out the other lights in the operating room (to eliminate extraneous light that would be visibly reflected from tissue in the body part under investigation) and shining the excitation light of near infrared wavelength into the body cavity or surgically created opening so that the fluorescent image received directly by the eye of the observer (e.g., the surgeon) is predominantly the fluorescent image emanating from the internalized NIR fluorophore in the field of vision. Light emanating from a source in a range between 600 nm and 900 nm, preferably between 650nm and 850nm, or between 1,000 and 1,750 nm would be used in accomplishing the goal of direct visualization by the observer so that light reflecting from the body part, other than that from the internalized NIR fluorophore, is minimized or eliminated. Now, with or without aid from any type of endoscopic device, the fluorescent image produced by the methods of the invention is such that it can be viewed without aid of an image processing device, such as a CCD camera, TV monitor, photon collecting device, and the like.
In disclosed methods of the invention, the potential tumoral tissue is "exposed" to the excitation light (e.g., by surgically created opening or endoscopic delivery of the light to an interior location). The methods of the invention are particularly suited to *in vivo* detection of tumoral tissue located at an interior site in the subject, such as within a natural body cavity or a surgically created opening, where the diseased tissue is "in plain view" (i.e., exposed to the human eye) to facilitate a procedure of diagnosis, biopsy or surgical excision of the area that has been highlighted by internalization of the conjugate's NIR fluorophore from the composition of the invention. As the precise location and/or surface area of the

tumoral tissue are readily determined by the internalization of the conjugate's NIR fluorophore, the methods employing the composition of the invention provide a valuable guide to the surgeon, who needs to "see" in real time the exact outlines, size, etc. of the mass to be detected or resected as the surgery proceeds.

These methods advantageously provide an improved method of performing image guided diagnosis and surgery on a subject as the administration of a composition comprising the conjugate disclosed previously under conditions and for a time sufficient for said conjugate to accumulate at a given surgical site will assist a surgeon in visualizing the tumoral tissue, potentially for removing said tissue.

The most suitable route for administration of the composition of the invention will vary depending upon the carcinoma state and/or location to be resected, or the location of the suspected condition or carcinoma to be diagnosed. For example, the composition of the invention may be administrated orally or parenterally.

Advantageously, the route of administration of the composition of the invention is parenteral. As used herein, the term "parenteral" includes intravenous, intramuscular, subcutaneous, or intraperitoneal administration.

In relation to EOC, the preferred route of administration of the composition of the invention is intraperitoneal.

It should be understood that in any of the methods of the invention, the composition of the present invention is administered before the step a) of illuminating the conjugate to visualize the near infrared fluorophore's conjugate using a corresponding infrared light.

Accordingly, the composition of the present invention is administered to the subject within a period of not less than about 12 hours before the step a) and, preferably, the day before the step a).

Surprisingly, the inventors have now established the A38C-ALEXA647 probe is still enabling to identify tumoral tissues 6 weeks after its administration to the subject. Thus, the composition of the invention is useful for diagnosing carcinoma corresponding to non-resected tumoral tissue in post-operative explorations of subject.

Accordingly, the composition of the invention can be use in a method of performing carcinoma image guided post-operative carcinoma diagnosis on a subject who has undergone a surgical resection of his tumoral tissues, preferably an image guided surgery using the composition of the invention, wherein the step b) is of performing non-resected carcinoma diagnosis when identifying non-resected tumoral tissues that fluoresce upon excitation by the infrared light, preferably wherein the subject has been administered with said composition within a period of more than about 1 week before the step a), and preferably within a period of more than about 3 weeks before the step a).

A "subject" as the term is used herein is contemplated to include any mammals, such as a domesticated pet, farm animal, or zoo animal, but preferably is a human.

The composition of the present invention is administered in an "effective amount" for performing image guided carcinoma diagnosis or surgery. An effective amount is the quantity of a conjugate necessary to aid in direct visualization of any tumoral tissue located in the body part under investigation in a subject. Amounts effective for diagnostic or surgery use will, of course, depend on the subject, on the size and location of the body part to be investigated, on the affinity of the conjugate for the tumoral tissue, on the type of tumoral tissue, as well as on the route of administration.

In a relation to a human subject, an effective amount of composition for administration corresponds to 0.01 to 100 mg of conjugate per kg of subject, preferably to 0.05 to 25 mg of conjugate per kg of subject, and most preferably to 0.1 to 1 mg of conjugate per kg of subject.

As discussed previously, the inventors were now able to increase considerably this detection threshold of their methods by identifying carcinoma nodules whose surface was as few as 50 $\mu m^2$. Because of this considerable increase in this detection threshold, the methods of the invention thus present an increased sensitivity. Accordingly, the risk of patients' relapses will be reduced, thus increasing the life expectancy of the patients.

According to still another preferred embodiment of the methods of the invention, the tumoral tissues are tumoral nodules whose surface is less than 10 000 $\mu m^2$ -i.e., 0.01 $mm^2$- corresponding to the detection threshold in COUSTETS *et al.* (abovementioned, 2020), preferably carcinoma nodules whose surface is equal or less than 100 $\mu m^2$; and preferably carcinoma nodules whose surface is around 50 $\mu m^2$.

In the following, the invention is described in more detail. Yet, no limitation of the invention is intended by the details of the examples. Rather, the invention pertains to any embodiment which comprises details which are not explicitly mentioned in the examples herein, but which the skilled person finds without undue effort.

## Examples

Conjugate preparation

A cDNA was cloned to obtain the following fusion protein: histidine tag, TEV site and the XCL variant A38C (SEQ ID NO:2). The histidine tag was added to facilitate the purification of the recombinant protein on an affinity column using nickel as ligand and the TEV site was added to eliminate the tag by incubation with TEV protease. The construction was placed downstream of the T7 promoter. E. coli BL21 (DE3) strain transformed with the plasmid including this construction was grown in rich medium until an OD of 1 was reached. T7 RNA polymerase mediated transcription was induced with 1

mM isopropyl-thiogalactopyranoside and the culture temperature was decreased to 18 °C. Cells were harvested by centrifugation, lysed by sonication and the protein was first purified over a Nichromatography column using 0.1 M imidazole in the eluting buffer.

A second purification step was then achieved by size exclusion chromatography using a SEPHACRYL S300 column (600 mm × 26 mm) equilibrated with buffer A (50 mM phosphate, 100 mM NaCl) pH 7.2 before a run at a 1.5 mL per minute flowrate.

Endotoxins detection and quantification of the purified solution were done using ENDOSAFE NEXTGEN-PTS (CHARLES RIVER) according to manufacturer's protocol. The results have shown a very strong level of endotoxins with more than millions IU (International Unit) per mL. The protein concentration of the solution was near 1mg/mL.

Accordingly, a third purification step for avoiding endotoxin contamination was achieved by anion-exchange chromatography (AEX) using SARTOBIND Q according to manufacturer's protocol.

The purified proteins were finally dialyzed against buffer A and concentrated on a VIVASPIN 15R column (SARTORIUS 10 000 MWCO).

After dialysis, the recombinant A38C protein (SEQ ID NO:1) was labeled with a near infrared emitting dye (ALEXA FLUOR 647), with an ALEXA FLUOR 647 NHS ester covalent bound to ε-amine lysine residues, using CF®647 SE PROTEIN LABELING KIT (cat n°#92218, Biotium, USA) according to manufacturer's protocol. Briefly, A38C (30 μM) were incubated overnight at room temperature in the dark in a medium composed of 100 mM sodium bicarbonate (pH 8.3) and CF™647 dye (BIOTIUM). After elimination of unconjugated free dye by gel filtration (BIOGEL P30, BIO-RAD), the labeled A38C protein was recovered, and the yield of labeling was determined by spectrophotometry.

The protein-bound dye ratio was calculated using the following molar extinction coefficient for CF™647: $\varepsilon_{650nm}$ = 240,000 $M^{-1}$ $cm^{-1}$. Protein concentrations after labeling were calculated using $\varepsilon_{280nm}$ = 119,640 $M^{-1}$ $cm^{-1}$ with a previous correction from the absorption of the fluorophore at this wavelength.

The typical labelling ratio was 1 mole of dye per mole of tetramer.

As determined in LADURANTIE et al. (Nanoscale, vol.11, p:3248-3260, 2019), the conjugate is a multimer, with a dynamic exchange between tetrameric and dimeric forms. In fact, the A38C-ALEXA647 tetramer can split into two dimers upon reduction of two disulfide bounds.

Finally, endotoxins detection and quantification of the final solution were done using ENDOSAFE NEXTGEN-PTS (CHARLES RIVER) according to manufacturer's protocol. The results have shown a low of endotoxins with less than 10 ill per mL of solution.. Again, the protein concentration of the solution was near 1mg/mL.

<u>Development of Patient-Derived Xenograft (PDX) to obtain a reliable animal model representative of human late stages epithelial ovarian cancers (i.e. ovarian peritoneal carcinomatosis):</u>

Patient-derived xenograft tumors are useful preclinical tools for drug discovery and assessment, since retaining molecular and histopathological features of the originating tumor.

Ascites were collected from EOC patients with different genetic profiles (wtBRCA/mBRCA, HRD/HRP) during an ongoing clinical trial (PlatinOv-NCT03954171) at ONCOPOLE CLAUDIUS REGAUD.

Female nude mice (BALB/c nu/nu from Janvier Labs, France), 8 weeks of age and weighing between 18 and 23 g, were used in this study. All animal experiments were conducted in accordance with ARRIVE guidelines, European Union Directive 2010/63/EU and French procedural guidelines for animal handling, with approval from the ethical committee (C2EA-01) of the Ministère de l'Enseignement Supérieur, de la Recherche et de l'Innovation (MESRI) (agreement number #2017080816182218). In the sequential procedure, nude mice were intraperitoneally injected with ascites. Now, very few implantations were observed. Thus, implantations were observed only with ascites from EOC patients with high-grade serous tumors. For these successful mice primary implantations, tumors and ascites development were typically observed between 60 to 90 days. Animals were anaesthetized using isoflurane inhalation anesthesia for collecting the ascites resulting of these successful mice primary implantations. Part of these collected ascites were injected into new animals for a first *in vivo* "passage", whereas the rest were preserved in liquid nitrogen. Finally, the histological non-derivation of the tumors collected from the animals as compared to the original patient's tumors was checked after three *in vivo* "passages". Mice were sacrificed when significant ascites production occurs -i.e., animal weight exceeds 20% of the initial weight of the animal, abdomen circumference >9.5cm, bloody ascites-.

Once PDX model was established and validated as histopathologically equivalent to the patient tumor, 0.5 mg of conjugate per kg of subject was administered by intraperitoneal injection to the animals the day before euthanasia. It should be noticed that no toxicity was observed with up to 25 mg of conjugate per kg of subject. After euthanasia, autopsies were performed under fluorescence monitoring. Macroscopy fluorescence imaging was carried out on an upright "MACROFLUO" fluorescence macroscope (LEICA Microsystems), equipped with a Cool Snap HQ2 Camera (ROPER SCIENTIFIC, PHOTOMETRICS) and a PLANAPO 0.5x, WD 187 mm objective. Tissues were imaged using Cy5 filter (excitation filter, BP: 620/60 nm, emission filter, BP:700/75 nm) in order to see the bio distribution of A38C-ALEXA647 conjugate thanks to the fluorescent signal emitted by the ALEXA647 moiety of the conjugate. Tissue-emitting near infrared

signals were collected and examined on histological frozen sections to ensure their tumorous origins. As a result, the efficiency of the conjugate to properly detect tumor tissues -i.e., microscopic tumor nodules- in the abdominal cavity of the PDX models, thanks to its polypeptide A38C moiety, was validated.

This fluorescence detection made it possible to establish the Signal to Noise Ratios (SNR) of the A38C-ALEXA647 probe, from the "endotoxin-free" production batches, on each group of peritoneal organs. Signal-to-noise ratios of the A38C-alexa647 probe were calculated by image analysis on each group of peritoneal organs and are listed in the following Table 1.

Table 1

| | Digestive tract | Spleen-large Omentum | Liver small Omentum | Diaphragm | Retroperitoneum |
|---|---|---|---|---|---|
| Signal to Noise Ratios | 7 | 9 | 8 | 11 | 14 |

The results have shown that the signal from the A38C-ALEXA647 probe persists for at least 6 weeks in the abdominal cavity of the animals. Moreover, the results confirm the animal model comprising patient-derived xenograft tumors as a valuable model. Now, the resulting SNRs vary from 7 to 14 depending on the implantation site of the nodules (See. Table 1). These SNRs are unexpectedly high as compared to the ones obtained in COUSTETS *et al.* with the same probe in an ovarian peritoneal carcinomatosis mouse model (abovementioned, 2020).

This first animal model was developed by the inventors for evaluating the probe's specificity and sensitivity. The IGROV cells express GFP constitutively and this signal was used to determine whether the animal model was relevant for the evaluation of the probe -i.e., whether the nodules developed following the injection of IGROV cells were in the microscopic range.

Now, the evaluation of nodules' surface, reported in figure 4B of COUSTETS *et al.* was made using the GFP constitutive signal of the IGROV cells intraperitoneally injected to induce peritoneal carcinomatosis. Once this model validated by verifying the presence of a majority of microscopic nodules (<1mm$^2$), two procedures have been used to evaluate the nodule detection efficiency of the probe. The first protocol, named sequential, consisted in an intraperitoneal injection of IGROV cells to induce peritoneal carcinomatosis and then, 3 weeks later, an intraperitoneal injection of the probe the day before euthanasia (figure 5A of COUSTETS *et al.*). The goal was to mimic an injection of the probe before cytoreductive surgery to use it as intraoperative assistance. In a second procedure, called coinjection, IGROV cells and the A38C-ALEXA647 probe were intraperitoneally simultaneously injected (figure 6A of COUSTETS *et al.*) to mimic what can happen in clinical situations. Indeed, during surgery, an iatrogenic spreading of tumor cells occurs in the abdominal cavity. These cells might re-implant and lead to relapses. This coinjection procedure was also used to analyse the persistence of the *in vivo* labelling of the probe through time. The probe's SNRs (Signal to Noise Ratios) taught in figure 5C and 6D of COUSTETS *et al.* was based on the ALEXA647 signal emitted by the fluorescent moiety of the probe. To evaluate SNRs on the fluorescence images of tumor-bearing mice, the mean fluorescence intensities (MFIs) of three regions of interest (ROI) per frame have been calculated. One ROI was located on the brightest NIR fluorescence point of the frame (ROI 1), the second on the darkest point inside the tissue (ROI 2) and the third on the frame out that surrounds the tissue (ROI 3). The SNR were calculated on three mice from each protocol according to the formula:

$$SNR = (ROI1\ x\ ROI3)/(ROI\ 2\ x\ ROI3)$$

The SNRs results for the IGROV model according to the two procedures are presented in table 2.

Table 2

| | Digestive tract | Spleen-large Omentum | Liver small Omentum | Diaphragm | Retroperitoneum |
|---|---|---|---|---|---|
| Signal to Noise Ratios in the coinjection procedure | 4 | 5 | 8 | 8 | 6 |
| Signal to Noise Ratios in the sequential procedure | 5 | 5 | 5 | 7 | 7 |

The results show that, as illustrated in the table 2, the measured ALEXA647 SNR is comprised between 4 and 7. If such SNRs were certainly interesting, those values positioned the A38C-ALEXA647 probe as a starting point among others for developing a further probe having the required sensitivity and specificity to be used in therapy. Accordingly, the PDX revealed unexpectedly high SNRs values of the A38C-ALEXA647 probe (See. Tables 1 and 2), with particular interest for tumoral tissues location in digestive tract, spleen/large omentum and retroperitoneum. In fact, the SNRs values of the newly purified A38C-ALEXA647 probe for EOC tumoral tissues located in the digestive tract, in the large omentum and in the retroperitoneum in this patients' derived xenograft (PDX) model corresponds to good sensitivity and specificity values with SNRs values of 7, 9 and 14 respectively, instead of the poor sensitivity and specificity SNRs values of 4, 5 and 6 respectively determined previously in the IGROV model.

In order to determine the probe sensitivity, the mean tumoral surface was determined for the experiments disclosed in COUSTETS *et al.* (abovementioned, 2020) and for the experiments realized in animal model comprising patient-derived xenograft tumors. For the IGROV animal model, ffluorescence images (GFP signal) of each carcinomatosis sites were processed using FIJI software. The images were first scaled according to calibration of the fluorescence macroscope camera and objective (mm/pixel, depending on the magnification used during acquisition). Then a threshold was applied to the images and the analyse particles function of the software was used to get the number of nodules and their area in $mm^2$. For the PDX animal model, fluorescence images were analysed in the same manner as described above but by using the ALEXA647 signal because patients' cells were not expressing GFPhe mean tumoral nodule surfaces depending on the body location and for both experiments' series are detailed in Table 3.

Table 3

| | Mean tumoral nodule surface ($mm^2$) | | | | |
|---|---|---|---|---|---|
| | Digestive tract | Spleen-large Omentum | Liver small Omentum | Diaphragm | Retroperitoneum |
| IGROV animal model | 0.194 | 0.137 | 0.261 | 0.209 | 0.306 |
| PDX animal model | 0.00585 | 0.01 | 0.03 | 0.021 | 0.328 |

Again, these results also confirm that the SNRs obtained with the newly purified probe are unexpectedly increased. In fact, the results show that the obtained detection sensitivity with the newly purified A38C-ALEXA647 probe was at least 10 to 30 times higher than previously estimated for this probe -i.e., detected nodules' surfaces were 10 to 30

times smaller in the PDX model as compared to the IGROV one. Knowing that the A38C-ALEXA647 probe allows the detection of microscopic tumor nodules, non-vascularized and invisible to the naked eye with a surface as few as 50$\mu m^2$, its use make possible to perform excision surgery of previously unidentified tumoral nodules.

In relation to the observed higher apparent affinity of the A38C-ALEXA647 probe, the authors notice that the conjugate binds to a saccharide, namely the disaccharide (β-D-Gal (1-3) D-GalNAc) corresponding to the Thomsen-Friedenreich antigen. Thus, it is expected that this conjugate interacts with other saccharides with approaching structures, potentially with a reduced affinity. In this way, endotoxins are lipopolysaccharides, and it was hypothesized that the conjugate interact with endotoxins thus corresponding to TF antigen competitors. Accordingly, a strong endotoxins concentration may have interfered in conjugate specific binding with TF antigen resulting in apparent reduced A38C-ALEXA647 probe sensitivity. Finally, the introduced further purification step, by eliminating endotoxins corresponding to TF antigen competitors, may have restored the normal binding kinetic of A38C-ALEXA647 probe specific binding to TF antigen resulting in a higher apparent affinity.

<u>Use of A38C-ALEXA647 probe for resection surgery of tumoral tissue in EOC patients:</u>

For its first-in-human trials, 0.5 mg of conjugate per kg of subject are administered to the patients by intraperitoneal injection the day before surgery -i.e., the day patients are hospitalised.

After standard cytoreductive surgery procedure under white light, the clinicians use the fluorescent camera equipment at disposal in the operating room to visualize the fluorescent signal, emitted by the fluorophore moiety of the conjugate, to reveal the otherwise unseen microscopic cancerous nodules within the abdominal cavity of the patients.

This real time visualization enables the surgeons to excise all tissues emitting near infrared fluorescence - i.e., all tumorous tissues specifically identified by A38C - in order to reach complete debulking and, by doing so, prevent relapses for those patients.

## Claims

1. A pharmaceutical composition comprising an imaging agent, eventually associated with a pharmaceutically acceptable carrier, said imaging agent consisting in a conjugate of:

    a) a polypeptide A38C having the amino acid sequence SEQ ID NO: 1, and
    b) a Near Infra-Red (NIR) fluorophore;

    **characterized in that** the endotoxin concentration of said pharmaceutical composition is less than 10 International Units (IU) of endotoxins per mL of composition, preferably less than 5 IU/mL, and most preferably less than 1 IU/mL.

2. The pharmaceutical composition of claim 1, wherein said composition does not comprise any other imaging agent.

3. The pharmaceutical composition according to any one of claim 1 or 2, wherein the NIR fluorophore is a fluorophore, whose absorption and emission wavelengths are both comprised between 600 and 900 nanometers.

4. The pharmaceutical composition according to any one of claims 1 to 3, wherein the NIR fluorophore is selected in the group comprising INDOCYNINE GREEN (ICG), IRDYE800CW, ZW-800-1, SO456, Sulfo-Cy5 and Sulfo-Cy5.5, BM104 and BM105 (the NHS-activated version of BM104), CH1055, Allophycocyanin (APC), NOVAFLUOR 660, Cyanine5 (Cy5), EFLUOR660, ALEXA FLUOR 647, NOVAFLUOR RED 685, NOVAFLUOR RED 690, ALEXA FLUOR 660, NOVAFLUOR RED 700, ALEXA FLUOR 680, NOVAFLUOR RED 710, ALEXA FLUOR 700, NOVAFLUOR RED 725, NOVAFLUOR RED 755, ALEXA FLUOR 750, APC-EFLUOR 780, squaraines, xanthene derivatives, and boron dipyrromethenes (BODIPYs) or aza-BODIPYs.; preferably the IR fluorophore is ALEXA FLUOR 647 dye.

5. The pharmaceutical composition of claim 4, wherein the NIR fluorophore is an acid stable NIR fluorophore, whose structure and fluorescence are both maintained at pH 4.5 to 5.0 as compared to pH 7.0 to 7.5.

6. The pharmaceutical composition according to any one of claims 1 to 5, wherein the A38C polypeptide is under a multimeric form corresponding to a tetramer.

7. The pharmaceutical composition according to any one of claims 1 to 6, wherein the conjugate refers to a covalent complex between:

a) a polypeptide A38C having the amino acid sequence SEQ ID NO: 1, and
b) a NIR fluorophore.

**8.** The pharmaceutical composition of claim 7, wherein the conjugate refers to a covalent complex between the A38C variant (SEQ ID NO:1) and the ALEXA FLUOR 647 dye.

**9.** A composition comprising the conjugate as defined in any one of claims 1 to 8 for use in a method of performing image guided surgery of tumoral tissues on a subject suffering from carcinoma comprising:

a) illuminating the conjugate to visualize the near infrared fluorophore's conjugate using a corresponding infrared light; and
b) performing surgical resection of the tumoral tissues that fluoresce upon excitation by the infrared light, wherein the subject has been administered with said composition under conditions and for a time sufficient for the conjugate to accumulate at a given surgical site.

**10.** A composition comprising the conjugate as defined in any one of claims 1 to 8 for use in a method of performing image guided carcinoma diagnosis on a subject comprising:

a) illuminating the conjugate to visualize the near infrared fluorophore's conjugate using a corresponding infrared light; and
b) performing carcinoma diagnosis, when identifying tumoral tissues that fluoresce upon excitation by the infrared light, wherein the subject has been administered with said composition under conditions and for a time sufficient for the conjugate to accumulate at a given surgical site.

**11.** The composition for use in a method of performing image guided carcinoma diagnosis on a subject according to claim 10, wherein said method is of performing image guided post-operative carcinoma diagnosis, wherein the subject has undergone a surgical resection of his tumoral tissues, preferably an image guided surgery using the composition as defined in any one of claims 1 to 8, wherein the step b) is of performing non-resected carcinoma diagnosis when identifying non-resected tumoral tissues that fluoresce upon excitation by the infrared light, preferably wherein the subject has been administered with said composition within a period of more than about 1 week before the step a), and preferably within a period of more than about 3 weeks before the step a).

**12.** The composition for use in a method of performing image guided surgery of tumoral tissues on a subject suffering from carcinoma according to claim 9 or in a method of performing carcinoma image guided carcinoma diagnosis on a subject according to claim 10, wherein said carcinoma expressed the Thomsen-Friedenreich antigen.

**13.** The composition for use in a method of performing image guided surgery of tumoral tissues on a subject suffering from carcinoma according to any one of claim 9 or in a method of performing image guided carcinoma diagnosis on a subject according to claim 10, wherein said carcinoma is an Epithelial Ovarian Cancer (EOC), preferably the tumoral tissues are EOC nodules located in the digestive tract, in the spleen/large omentum, and/or in the retroperitoneum.

**14.** The composition for use in a method of performing image guided surgery of tumoral tissues on a subject suffering from carcinoma according to any one of claim 9 or in a method of performing image guided carcinoma diagnosis on a subject according to claim 10, wherein the subject has not been administrated with any other imaging agent.

**15.** The composition for use in a method of performing image guided surgery of tumoral tissues on a subject suffering from carcinoma according to any one of claim 9 or in a method of performing image guided carcinoma diagnosis on a subject according to claim 10, wherein the tumoral tissues are tumoral nodules whose surface is less than 1 0000 $\mu m^2$ -i.e., 0.01 $mm^2$-, preferably carcinoma nodules whose surface is equal or less than 100 $\mu m^2$; and preferably carcinoma nodules whose surface is around 50 $\mu m^2$.

Europäisches Patentamt
European Patent Office
Office européen des brevets

# EUROPEAN SEARCH REPORT

Application Number
EP 24 17 5611

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Coustets Mathilde: "La lectine de Xerocomellus Chrysenteron, un nano-objet théranostique pour l'imagerie et le traitement des cancers épithéliaux : preuve de concept appliquée aux carcinoses péritonéales d'origine ovarienne", , 7 September 2021 (2021-09-07), XP093215535, Retrieved from the Internet: URL:https://theses.hal.science/tel-03336575 | 1-15 | INV. A61K49/00 |
| Y | * pages 200-203, particularly page 200, first paragraph, last part and page 202, first paragraph, lines 3 to 5 * | 1-15 | |
| | ----- | | |
| Y | COUSTETS MATHILDE ET AL: "Development of a near infrared protein nanoprobe targeting Thomsen-Friedenreich antigen for intraoperative detection of submillimeter nodules in an ovarian peritoneal carcinomatosis mouse model", BIOMATERIALS, ELSEVIER, AMSTERDAM, NL, vol. 241, 22 February 2020 (2020-02-22), XP086080466, ISSN: 0142-9612, DOI: 10.1016/J.BIOMATERIALS.2020.119908 [retrieved on 2020-02-22] * "Results" section * | 1-15 | |
| | ----- | | |
| | -/-- | | |

| TECHNICAL FIELDS SEARCHED (IPC) |
|---|
| A61K |

The present search report has been drawn up for all claims

1

| Place of search | Date of completion of the search | Examiner |
|---|---|---|
| The Hague | 17 October 2024 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 17 5611

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LADURANTIE CAROLINE ET AL: "A protein nanocontainer targeting epithelial cancers: rational engineering, biochemical characterization, drug loading and cell delivery", NANOSCALE, vol. 11, no. 7, 14 February 2019 (2019-02-14), pages 3248-3260, XP093215845, United Kingdom ISSN: 2040-3364, DOI: 10.1039/C8NR10249J Retrieved from the Internet: URL:https://pubs.rsc.org/en/content/articlepdf/2019/nr/c8nr10249j> * "Results" section * ----- | 1-15 | |

TECHNICAL FIELDS SEARCHED (IPC)

The present search report has been drawn up for all claims

| Place of search | Date of completion of the search | Examiner |
|---|---|---|
| The Hague | 17 October 2024 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5730969 A


### Non-patent literature cited in the description


- **MANGEOLLE et al.** *Nanomaterials*, 2018, vol. 8 (8), 572
- **COUSTETS et al.** *Biomaterial*, 2020, vol. 241, 119908
- **LADURANTIE et al.** *Nanoscale*, 2019, vol. 11, 3248-3260
- **LADURANTIE et al.** *Nanoscale*, 2019, vol. 1 (1), 3248-3260
- **SPRINGER**. *J. Mol. Med.,*, 1997, vol. 75, 594-602